(19)

Europäisches
Patentamt
European
Patent Office
Office européen
des brevets

(11)  **EP 1 762 273 A2**

(12)  **EUROPEAN PATENT APPLICATION**

(43) Date of publication:
14.03.2007 Bulletin 2007/11

(51) Int Cl.:
*A61Q 19/00* (2006.01)    *A61K 8/86* (2006.01)

(21) Application number: 06018434.8

(22) Date of filing: 04.09.2006

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: 08.09.2005 IT MI20051658

(71) Applicant: **Solvay Solexis S.p.A.**
**20121 Milano (IT)**

(72) Inventor: **Pantini, Giovanni**
**20131 Milano (IT)**

(74) Representative: **Sama, Daniele**
**Sama Patents,**
**Via G.B. Morgagni, 2**
**20129 Milano (IT)**

(54)    **Cosmetic peeling compositions**

(57)    Use, for obtaining the cutaneous peeling of skin, of formulations comprising a component A) (per)fluoropolyether phosphate of general formula:

$$R_f\text{-}[CF_2CH_2\text{-}O\text{-}L\text{-}P\,(O)\,(OZ_1)\,(OZ_2)]_1 \qquad (I)$$

wherein l, L, $Z_1$, $Z_2$, $R_f$ are as defined in the application.

EP 1 762 273 A2

**Description**

The present invention relates to the use of compositions containing specific perfluoropolyether derivatives for cutaneous treatments of skin peeling which allow to obtain a smoother skin. From the cosmetic point of view this treatment produces an antiwrinkle and antiageing effect.

The compositions of the present invention can also be used for treating cutaneous phenomena due to skin hyperkeratinization. The latter phenomenon brings to the formation on the skin of a stratum corneum more thickened than the normal one. This phenomenon can be physiological, for example deriving from the slowdown of the cutaneous cellular renewal owing to ageing, or it can casually arise as for example in case of cicatrix or sun stain formation.

It is known in the prior art that, in cosmetic treatments to obtain cutaneous peeling, compositions are used containing as active ingredients compounds having at least one acid group mainly selected from the following classes:

- alpha hydroxyacids, monocarboxylic as, for example, glycolic acid and lactic acid, and di- and polycarboxylic as, for example, malic acid, tartaric acid, citric acid;
- beta hydroxyacids as, for example, salicylic acid;
- ketoacids as, for example, pyruvic acid;
- poly-alpha-hydroxyacids as, for example, polylactic acid;
- non hydroxylated carboxylic organic acids having a short chain as, for example, trichloroacetic acid.

When on the skin hyperkeratinization phenomena are present, it is necessary that the cutaneous peeling treatment deeply acts so as to remove as much as possible or completely the stratum corneum more thickened than the normal one which has formed. In these cases compositions are applied in the form of aqueous solutions having high concentrations of the above active ingredients. For example, in case of compositions containing glycolic acid, the concentrations used are in the range 30%-70% by weight. The residence time of the preparation on the skin is critical, since, as said, a quick epidermis peeling is required, but at the same time the cutaneous irritation phenomena which can easily arise, due to the high concentration of the active ingredient, must be kept under control, and therefore limited as much as possible. For this reason these applications for cosmetic purposes using glycolic acid at the above high concentrations, generally in the range 30%-70% by weight, are carried out by the dermatologist.

However cosmetic formulations are also available in the market, wherein the concentration of the compounds having a peeling activity is lower, usually less than 10% by weight, and the pH of the formulations is buffered at values generally between 3.5 and 4.5. Therefore, in these compositions the compound having at least one acid group is present in a partially salified form. With these compositions, daily and continued treatments, even for weeks, can be performed. These cosmetic formulations are usually utilized for the cutaneous cleaning or as antiwrinkle preparations. In the former case they are in the form of gels and emulsions. The latter are marketed, depending on their viscosity, under the form of milk, lotions and creams. The drawback of these compositions is that the peeling effectiveness is very limited. On the other hand, if the pH is lowered towards acid values, as said, there are cutaneous irritation effects. The need was felt to have available compositions for topical applications having the following combination of properties:

- improved peeling activity in comparison with that obtainable by using the commercial compositions containing the same amount by weight of the acid compounds used in the prior art as peeling agents;
- cutaneous irritation side effects substantially absent.

The Applicant has surprisingly and unexpectedly found that it is possible to solve the above technical problem by using compositions containing the active ingredients described hereunder.

An object of the present invention is the use to obtain cutaneous peeling of formulations comprising as peeling component the following component A):

(per)fluoropolyether phosphate of general formula:

$$R_f\text{-}[CF_2CH_2\text{-}O\text{-}L\text{-}P(O)(OZ_1)(OZ_2)]_l \qquad \text{(I)}$$

wherein $l = 1$ or 2;

- L is a bivalent linking group, preferably of the $(CHR_1CHR_2O)_n$ type wherein $R_1$, $R_2$ equal to or different from each other, are selected from H, $CH_3$; n is an integer between 1 and 50, preferably between 1 and 6;
- $Z_1$, equal to or different from $Z_2$, is selected from H, alkaline or ammonium cation, mono- di- or tri-alkanolammonium cation wherein the alkanol comprises from 1 to 20 C atoms, preferably 2-6 C atoms, di- or tri- or tetra-alkylammonium cation wherein the alkyl comprises from 1 to 20 C atoms, preferably 2-6 C atoms, or

$R_f$-(CF$_2$CH$_2$-O-L-;

- $R_f$ represents a (per)fluoropolyether chain with a free valence when l = 1 and two free valences when l = 2, said (per)fluoropolyether chain having number average molecular weight in the range from about 400 to about 3,500, preferably from 800 to 2,000, said (per)fluoropolyether chain comprising repeating units selected from one or more of the following:

a) -(C$_3$F$_6$O)-;
b) -(CF$_2$CF$_2$O)-;
c) -(CFL$_0$O) -, wherein L$_0$ = -F, -CF$_3$;
d) -CF$_2$(CF$_2$)$_{z'}$CF$_2$O-, wherein z' is an integer 1 or 2;
e) -CH$_2$CF$_2$CF$_2$O-.

When $R_f$ is monofunctional (l = 1), it has one end group of the perfluoroalkyl type as, for example, CF$_3$O-, C$_2$F$_5$O-, C$_3$F$_7$O-; optionally in perfluoroalkyl end groups one fluorine atom can be substituted by one chlorine or hydrogen atom.

The preferred compound of general formula (I) is the one wherein Z$_1$ or Z$_2$ are different from $R_f$-[-CF$_2$CH$_2$-O-L-; preferably Z$_1$ = Z$_2$ = H and l = 2.

In particular $R_f$ is a substituent of bifunctional (per)fluoropolyether type and preferably has one of the following structures:

1) -(CF$_2$O)$_a$-(CF$_2$CF$_2$O)$_b$-
a and b being integers such to give the above number average molecular weight, b/a being between 0.3 and 10, extremes included, a being different from 0;
2) -(CF$_2$-(CF$_2$)$_{z'}$-CF$_2$O)$_{b'}$-
b' being an integer and such to give the above number average molecular weight, z' being an integer and equal to 1 or 2;
3) -(C$_3$F$_6$O)$_r$-(C$_2$F$_4$O)$_b$-(CFL$_0$O)$_t$-,
r, b and t being integers such to give the above number average molecular weight, r/b = 0.5-2.0 when b is different from 0, (r+b)/t = 10-30, t being different from 0;
4) -(OC$_3$F$_6$)$_r$-(CFL$_0$O)$_t$-OCF$_2$-R'$_f$-CF$_2$O-(C$_3$F$_6$O)$_r$-(CFL$_0$O)$_t$-
r and t being integers as above such to give the above number average molecular weight;
5) -(CF$_2$CF$_2$CH$_2$O)$_{q'}$-R'$_f$-O-(CH$_2$CF$_2$CF$_2$O)$_{q'}$-
wherein:

q' is an integer such to give the above number average molecular weight;
R'$_f$ is a fluoroalkylene group from 1 to 4 carbon atoms;
L$_0$ is selected from F, CF$_3$;

6) -(C$_3$F$_6$O)$_r$-OCF$_2$-R'$_f$-CF$_2$O-(C$_3$F$_6$O)$_r$-
wherein r is an integer such to give the above number average molecular weight and R'$_f$ is as above.
In said formulas:

the unit -(C$_3$F$_6$O)- can represent units of formula:

-(CF(CF$_3$)CF$_2$O)- and/or -(CF$_2$-CF(CF$_3$)O)-.

In formula (I) the preferred (per)fluoropolyether chain $R_f$ is selected from the following structures:

from those bifunctional (when l = 2):

-(CF$_2$O)$_a$-(CF$_2$CF$_2$O)$_b$-;
-(C$_3$F$_6$O)$_r$-(C$_2$F$_4$O)$_b$-(CFL$_0$O)$_t$-;

still more preferably
$R_f$ = -(CF$_2$O)$_a$-(CF$_2$CF$_2$O)$_b$- (l = 2);
from those monofunctional (when l = 1):

- $(C_3F_6O)_r$-$(CFL_0O)_t$-;

wherein $L_0$ and the a, b, r, t indexes have the above value;

The compounds of formula (I) preferably used according to the present invention are those wherein L = $(CH_2-CH_2O)_n$ with n integer from 1 to 4; $Z_1$, equal to or different from $Z_2$, is selected from H, $NH_4$, or an alkaline metal cation; 1 = 2.

The compounds, according to the geneal formula (I), having the following formulas, are still more preferred:

$$CF_3-O(CF_2CF(CF_3)O)_r(CF_2O)_a-CF_2-CH_2(OCH_2CH_2)_nO-PO(OH)_2 \qquad (II)$$

wherein r/a = 0.5-2.0 (a being different from 0) and n = 1-2;

$$[-O(CF_2CF_2O)_b(CF_2O)_a-] [-CF_2-CH_2-(OCH_2CH_2)_nO-PO(OH)_2]_2 \qquad (III)$$

wherein b/a = 0.5-3.0 (a being different from 0) and n = 1-2;
wherein a, b and r have the above meaning.

The (per)fluoropolyethers of general formula (I) are obtainable by the well known processes of the prior art, see for example the following patents herein incorporated by reference: US 3,665,041, US 2,242,218, US 3,715,378 and the European patent EP 239,123. The functionalized fluoropolyethers having an hydroxyl termination are obtained for example according to EP 148,482, USP 3,810,874.

The preparation of the monofunctional (per) fluoropolyether phosphates of general formula (I) wherein $R_f$ has one perfluoroalkyl end group can be carried out by reacting the corresponding (per)fluoroalkylenoxides monohydroxy-ended with $POCl_3$. A molar ratio $POCl_3$/hydroxy-ended compound between 2/1 and 10/1, preferably between 6/1 and 8/1, is used. The reaction is carried out by slowly dripping the mono-hydroxy-ended (per)fluoropolyether in $POCl_3$, at a temperature between 50°C and 100°C, preferably between 70°C and 80°C, by eliminating the HCl vapours in a KOH trap. The $POCl_3$ excess is removed by distillation while the formed adduct is hydrolyzed by $H_2O$. The hydrolyzed adduct is further reacted, for example with an equimolar amount of hydroxy-ended (per)fluoropolyether compound to form the monoester.

The separation of the obtained product takes place by extraction with a suitable organic solvent as, for example, ethyl acetate. From the organic phase the product of formula (I) is separated according to known techniques, for example by solvent evaporation.

The preparation of the bifunctional (per)fluoropolyether phosphates (in this case $R_f$ of formula (I) does not have a perfluoroalkyl end group) can be carried out by reacting the corresponding (per)fluoroalkylenoxides di-hydroxy-ended with $POCl_3$. A molar ratio $POCl_3$/di-hydroxy-ended compound between 4/1 and 20/1, preferably between 12/1 and 16/1, is used. The reaction is carried out by slowly dripping the hydroxy-ended compound in $POCl_3$, at a temperature between 50°C and 100°C, preferably between 70°C and 80°C, by eliminating the HCl vapours in a KOH trap. The $POCl_3$ excess is removed by distillation while the formed adduct is hydrolyzed by $H_2O$. The separation of the product takes place by extraction with an organic solvent as, for example, ethyl acetate. From the organic phase the product is separated according to known techniques, for example by solvent evaporation.

The concentration of component A) in the formulations of the present invention is between 0.1% and 30% by weight, preferably between 1% and 10%.

The peeling compositions of the invention comprise component A) solublized in water (component C)) or in a polar organic solvent (component B)), or their mixtures.

Among polar organic solvents it can be mentioned:

- linear or branched when possible alcohols, from 2 to 3 carbon atoms and their ethers, preferably methylic alcohols;
- linear or branched glycols from 2 to 6 carbon atoms; optionally mono alkyletherified wherein the alkyl group, linear or branched, is $C_1$-$C_4$;
- ethers as, for example, dimethoxymethane;
- esters of acids and alcohols having, respectively, from 1 to 4 carbon atoms and liquid at room temperature; or respective mixtures.

The water (component C)) can be used alone or in admixture with a solvent B).

When only water (component C)) for preparing the solution of A) in C), is used, a basic component as defined below is added to salify at least 60% of the acid groups present and to obtain a final pH not lower than 4.

When mixtures of water with at least a solvent B) are used for preparing the solution of A) in the mixture of C) and B), preferably component A) is dissolved in solvent B) before adding water (component C)).

The peeling formulations of the invention can preferably be used in the form of gels and emulsions. The latter can be, depending on their fluidity and viscosity, under the form of milk, lotions, creams.

Optional additive components of the formulations of the present invention are those of the commercial cosmetic compositions. Emollient-, emulsifying-, viscosifying-, hydrating-, preserving-, anti-ageing agents as, for example, sun filters as UV filters, antioxidant agents to block the free radicals (the so called scavengers), for example vitamin C and polyphenols; peeling agents of prior art, preferably hydroxylated, as those indicated hereunder, can be mentioned.

The compositions of the present invention can be prepared with the known methods of the prior art.

Preferably said compositions are prepared by dissolving component A) in a solvent or mixture of solvents comprising B) or C) or their mixtures, in case by adding a basic component as defined below, and by optional addition of further components and/or excipients of the peeling formulations as above, obtaining the percentage by weight of component A) comprised in the above limits.

More preferably the prepartion is carried out by using a starting solution of component A) in component B) and/or C). In the latter case also a basic component is added and then one dilutes by the addition of B) and/or C) and optionally of other components and/or excipients, liquid or solid, of the formulation.

A particularly preferred process for preparing the most effective formulations resides in using water (component c)) to prepare the solution of component A), then adding a basic component to salify at least 60% of the acid groups (of A) and obtain a final pH not lower than 4.

Another process which can be used to obtain the formulations of the invention having more acid pHs (lower than 4) comprises the use of mixtures of water component C) with at least one solvent B) to dissolve component A). In this case the salification of component A) is optional when component A) is dissolved in solvent B) before adding component C).

The solutions of the invention comprising A) and C), optionally B), are limpid and in practice colourless. From these solutions gels and the various kinds of emulsion can be prepared as said above.

By using the above processes, compositions having a wide pH range, for example from pH 2 to pH 9, preferably from pH 3 to pH 7 can be obtained.

It has been in fact unexpectedly and surprisingly found by the Applicant that it is possible to use formulations according to the present invention having even very acid pH, for example pH 2.5, without undesired cutaneous effects, such as cutaneous irritation. This is surprising as the peeling agents known in the prior art have a much more irritating effect, the pH being equal. This result is surprising and unexpected as the skilled man in the field would have deduced that the formulations of the invention used with acid pHs would have had the irritating effects typical of the formulations of the prior art.

Component B) is preferably selected from the following: ethanol, ehtylene glycol, isopropanol, propanol, acetone, methoxyethanol, propylen glycol, propan-1,2-diol, dimethoxymethane, methoxy-isopropanol, diethylen glycol, butan-1,4-diol, diethylenglycolmonoethylenether, pentan-1,2-diol, diethylenglycol monoethylether, dipropylenglycol, dipropylenglycol monomethylether, dipropylenglycol monoethylether; still more preferably: ethanol, pentan-1,2-diol.

When the starting solution contains components A) and B), the amount of A) ranges from 1% to 50% by weight, B) being the complement to 100% by weight.

Preferably, when the components of the starting solution are A) and C), the solution is prepared by suspending the perfluoropolyether phosphate, under stirring, in the water component C), by adding a solution of a basic component, for example, the base of an alkaline metal (sodium or potassium hydroxide), of the ammonium ion or of an organic base, preferably tertiary. The organic bases usable in cosmetics are well known. See for example International Cosmetic Ingredient Dictionary and Handbook, 7th ed. 1997, The Cosmetic, Toiletry, and Fragrance Association publisher.

In this way the hydrosoluble salts of component A) can be obtained. The pH of the obtained formulation in water has a value higher than or equal to 4, preferably comprised between 5 and 7. The amount of component A) in the starting solution of A) + C) ranges from 1% to 50% by weight, B) being the complement to 100% by weight.

When the components of the starting solution are A), B) and C), the amount of A) ranges from 1% to 50% by weight; B) ranges from 10% to 80%, component C) being the complement to 100% by weight of the solution, the amount of C) being lower than that of B). Preferably, the percentage by weight of component A) in the starting solution ranges from 20% to 40% by weight, component B) from 30% to 70% by weight and component C) in the required amount to obtain a limpid solution, which generally is between 5% and 30% by weight, the sum of the components being 100%.

The starting solution comprising the three components A), B) and C) is preferably prepared with a process comprising the following steps:

- solubilization, or dispersion with partial solubilization, of a (per)fluoropolyether phosphate component A) in the component B) at room temperature, generally between 15°C and 30°C, preferably under stirring;
- addition, preferably under stirring, to the mixture of component C) water, initially dropwise, so that component A) does not separate, each time dispersing the drop so as to restore the starting appearance of the solution, before adding further portions of water, which can then be gradually increased until completing the addition, obtaining at the end a limpid solution.

Preferably component C) is added at a temperature in the range from about 40°C to about 90°C, preferably from about 80°C to about 90°C.

As said, the solutions prepared with the processes illustrated above, can be then diluted, for example by using water and/or component B), optionally by adding other components and excipients, liquid or solid, to prepare a formulation wherein the concentration of component A) is within the above limits.

Preferably for the use according to the present invention the compositions containing component A) do not contain surfactants/emulsifiers and are prepared in the form of gel, by using as gelling agents natural and synthetic hydrophilic polymers, also in the form of the corresponding salts.

As said, it is however possible to use formulations under the form of emulsions as, for example, creams. In this case surfactants/emulsifiers can optionally be added. The amounts of these compounds are however very low and generally in the range from 0% to 5% by weight on the whole composition.

It has indeed been found by the Applicant that the perfluoropolyether phosphate component A), once solubilized in aqueous environment, has emulsifying properties in acid and neutral environment.

As said, to the formulations of the present invention emollient substances as, for example, mineral oils or fat acid esters, viscosifying, hydrating agents, etc. can also be added.

As said, it is also possible to use component A) in admixture with one or more compounds having a peeling activity of the prior art.

Preferably the latter are selected from alpha hydroxyacids, beta hydroxyacids, ketoacids, poly-alpha-hydroxyacids as, for example, the polylactic acid; and non hydroxylated carboxylic organic acids having a short chain $C_2$-$C_4$; retinol. Said compounds of the prior art having peeling activity are preferably selected from the following: lactic acid, glycolic acid, salicylic acid, trichloroacetic acid, acetic acid, hydroxyacetic acid, piruvic acid, citric acid, maleic acid, malic acid, tartaric acid, ascorbic acid, polylactic acid, hydroxyoctanoic acid, tartronic acid or hydroxypropandioic acid, glyceric acid or dihydroxypropionic acid, gluconic acid, glucuronic acid, mandelic acid, benzylic acid, 2-hydroxybutyric acid.

By carrying out several applications with the compositions of the present ivnention it is possible to obtain and maintain a smooth and more elastic skin. Therefore with the formulations of the invention an anti-wrinke effect is obtained with clear aesthetic benefits.

It has been surprisingly and unexpectedly found by the Applicant that the compositions of the present invention, the concentration of component A) being equal, result more effective when the formulation has an almost neutral pH, for example higher than 4, rather than towards values lower than said pH. This is extremely advantageous from the practical point of view since a cutaneous peeling treatment can be carried out without local irritation phenomena, the treatment being carried out at physiological pH.

It is however possible to use the compositions of the present invention, even at a relatively high concentration and even at very acid pHs, for example pH 2, as it has been found by the Applicant that the cutaneous irritation phenomena are very reduced or substantially absent.

Therefore the compositions of the present invention allow to carry out cutaneous peeling treatments, or substantial elimination of local cutaneous hyperkeratinization phenomena in a wide range of pH without substantially having cutaneous irritation effects.

As said, the peeling activity of the compositions of the present invention allows to use the same compositions to treat cutaneous hyperkeratinization phenomena. It is known indeed that compounds having a cutaneous peeling activity are active also in the cases of skin hyperkeratinization. See for example E.J. Van Scott et Al. "Hyperkeratinization, Corneocyte Cohesion, and Alpha Hydroxy Acids", J. Am. Acad. Dermatol. 11, 867-879, 1984; E.J. Van Scott "Control of Keratinization with $\alpha$ Hydroxy Acids and related Compounds", Acta Dermatol. vol. 110, October 1994, 586-590.

It has been found by the Applicant that the applications with the formulations of the present invention to treat the cutaneous hyperkeratinization are effective at concentrations lower than those of the prior art, without substantial effects of cutaneous irritation.

The use of the formulations according to the present invention is carried out by topical application, for example by letting the composition in loco for at least 24 hours, then repeating the application. The treatment can also be prolonged in the time, even for months, for example 6 months.

The following Examples are for illustrative purposes and do not limit the scope of the patent.

**EXAMPLES**

METHODS

Method to determine by the dansyl chloride the per cent increase of the peeling in vivo (peeling effect) induced by the application of compositions for topical use

The used method has been described in the literature by Marks R. et Al., J. Dermatol., 111, 265-270, 1984; B.D. Ridge et Al., J. Dermatol., 118, 164-174, 1988)

On the forearm of volunteers sites of application are identified having a circular shape with a diameter of 1.5 cm, in a number equal to the preparations to be tested plus an other site, having the same sizes, which is not treated with the compound having a peeling activity and therefore acts as a control. The perimeter of each site is indicated by pencil or by a marking pen.

On each site 0.2 ml of white paraffin containing 5% of dansyl chloride (Sigma Chemicals Co. Ltd., St. Louis, USA) are applied. Each site is covered with plasters of "Hill Top Chanbers" type (Hill Top Research, Inc., USA). After 24 ore the plasters are removed, the paraffin excess is removed. The dansyl chloride stain which remains on the site, being fluorescent, is detectable with a UV-visible lamp (Vilber Lourmat, France), by using a wave length of 365 nm. The fluorescence degree corresponding to the zero time of the experiment is evaluated, ascribing a score on the basis of the following scale:

0   absence of fluorescence in the site;
1   the fluorescent area is in per cent lower than 50% of the site area;
2   the fluorescence is diffused on about 50% of the site and the reminder of the area is not fluorescent;
3   the fluorescence is diffused on a large part of the site and there is a reduced area wherein the fluorescence is of an intensity clearly lower than that of the remainder of the site or can show no fluorescence;
4   total fluorescence on the whole surface of the site.

To the cutaneous sites 0.2 ml of a preparation in the gel formulation are then applied, in the evening, by using a gloved finger.

The experiment lasted 30 days and 10 readings have been carried out in all, one every three days.

The per cent increase of the cutaneous peeling is calculated with the following formula, wherein the expression "fluor degree" indicates the fluorescence degree evaluated with the score scale illustrated above:

$$\frac{(\text{fluor degree.}_{\text{untreated site}} - \text{fluor degree.}_{\text{considered site}}) \times 100}{\text{fluor degree.}_{\text{untreated site}}}$$

The test protocol requires that the test is interrupted when in a group of readings one site shows absence of fluorescence (score = 0).

Viscosity determination

The viscosity is determined with a Brookfield viscometer, rotor 29, rate 10 rpm, temperature 25°C.

Test of cutaneous irritation in vivo in occlusive conditions (patch test) on volunteers

The composition to be tested is applied on the volar part of the forearm of 20 volunteers, excluding subjects affected by dermatitis or with an anamnesis of allergic cutaneous reactions and subjects under treatment with antiinflammatory drugs.

When the tested formulation is under the form of hydrogel or emulsion, for the cutaneous application it is conveyed in devices "aluminium Fin chambers" (Bracco S.p.A., Milan, Italy) by a syringe, until completely filling the device, which is applied and left on the skin for 48 hours.

In case of formulations under the form of aqueous solutions, they are left absorb on discs of filter absorbing paper Whatmann, having a surface size like that of the above mentioned devices.

The evaluation of the cutaneous irritation (erythema) has been carried out, respectively, after 15 minutes and after 24 hours the removal from the skin of the device or of the paper disc Whatmann, according to the following arbitrary scale:

0     cutaneous irritation absent;
1     very light cutaneous irritation, poorly visible;
2     cutaneous irritation clearly visible;
3     moderate cutaneous irritation;
4     strong cutaneous irritation.

The scores obtained on the single volunteers are summed and averaged on the total number of the volunteers, thus obtaining the "Mean Irritation Index". The mean irritation index, on the basis of the obtained value, is then classified as follows:

| Value of the mean irritation index | Classification |
| --- | --- |
| <0.5 | the composition is not irritating |
| from 0.5 to 2 | the composition is slightly irritating |
| from 2 to 5 | the composition is moderately irritating. |

Determination in vivo of the Erythema Value

The volar part of the forearm of 20 volunteers having the same age, by a pen 8 circular sites (2 cm diameter) are marked. On two of said sites each formulation (0.2 ml) is applied. The used formulations in this experiment see herein below example 12, were those of exs. 8, 9 and 10 comparative, + a gel blank, not containing the active ingredient, but having the same compositions as for the other components. Gel application was made for each composition, twice a day, at 12 hours interval.

By a Mexameter MX18 instrument (Courage & Khazaka, Cologne, Germany) the Erythema Index has been measured. The instrument provides in fact a direct reading of said paramenter.

Said determinations were carried out on each site for each volunteer's forearm, before the first application (Day 0), then after 14 and 21 days, respectively. The experiment lasted 21 days, which should correspond to the mean period of a peeling treatment.

Determinations by the Mexameter MX18 instrument were taken in a room at a temperature of $27 \pm 2°C$ and moisture of 45-50%, wherein the volunteer was allowed to enter 15 minutes before the reading.

The readings for each composition applied were averaged on each volunteer and mean values $\pm$ S.D. were calculated and then the corresponding average values on the group were drawn.

The Erythema Values (EV) were calculated from the Erythema Index (EI) by using the following formula:

$$EV = \frac{EI_{gel\ under\ test} - EI_{gel\ blank}}{EI_{gel\ blank}} \times 100$$

EXAMPLE 1

Preparation of a composition for topical use in the gel form containing 5% of perfluoropolyether phosphate acid

A determined amount of bifunctional perfluoropolyether phosphate acid (PFPE phosphate) (Fomblin® HC/P2-1000), having number average molecular weight 2,500 in acid form, is dissolved at room temperature and under stirring in ethanol, then it is diluted with water and a viscosifying agent (xanthan rubber, Rhodicare T, Rhodia SA, France) and then an hydrosoluble dyestuff (green CI 42051) is added obtaining a gel having the following composition, as per cent by weight:

| | |
| --- | --- |
| Perfluoropolyether phosphate acid (Fomblin® HC/P2-1000) | 5.0 |
| Ethanol | 25.0 |
| Xanthan rubber (Rodicare® T by Rhodia) | 1.5 |
| Dyestuff (green CI 42051) | as it suffices |
| Water up to | 100 |

The obtained gel has a viscosity of 17,800 mPa.s and pH = 2.9.

EXAMPLE 2

Preparation of a composition for topical use under the gel form containing 5% of perfluoropolyether phosphate sodium salt

The Example 1 is repeated but by neutralizing with NaOH (18% by weight in water) the acid dissolved in the hydroalcoholic solution obtained by diluting the alcohol with water to prepare the formulation, by adding then an acrylate crosslinked polymer C/10-30 alkylacrylate (name INCI Acrylates C/10-30 Alkyl Acrylate Cross Polymer) Carbopol® Ultrez-21 (Noveon®, USA) and by adding an aliquot of the above NaOH solution up to a pH 5.6 of the formulation. The added dyestuff is blue CI 42080.
A gel having the following composition, as per cent by weight, is obtained:

| | |
|---|---|
| Perfluoropolyether phosphate acid (Fomblin® HC/P2-1000) | 5.0 |
| Ethanol | 25.0 |
| Sodium hydroxide (18% w/v in water) | 1.0 |
| Acrylate crosslinked polymer C/10-30 alkylacrylate | 1.2 |
| Sodium hydroxide (18% w/v in water) | as it suff. pH 5.6 |
| Dyestuff (blue CI 42080) | as it suffices |
| Water up to | 100 |

The obtained gel has a viscosity of 18,000 mPa.s and pH = 5.6.

EXAMPLE 3 (comparative)

Preparation of a gel containing 5% of glycolic acid

By starting from a solution of glycolic acid in water having titre 70%, it is diluted and an amount of ethyl alcohol and a viscosifying agent (xanthan rubber, Rhodicare T, Rhodia SA, France) and a water-soluble dyestuff (yellow CI 19140) is added, obtaining a gel having the following composition, as per cent by weight:

| | |
|---|---|
| Glycolic acid | 5.0 |
| Ethanol | 25.0 |
| Xanthan rubber (Rodicare® T by Rhodia) | 1.5 |
| Sodium hydroxide (18% w/v in water) | as it suff. pH 3.0 |
| Dyestuff (yellow CI 19140) | as it suffices |
| Water up to | 100 |

The obtained gel has a viscosity of 20,000 mPa.s.

EXAMPLE 3A (comparative)

A gel without any active peeling ingredient is prepared (reference control) having the following composition, in per cent by weight:

| | |
|---|---|
| Ethanol | 25.0 |
| Acrylate crosslinked polymer C/10-30 alkylacrylate | 1.2 |
| Sodium hydroxide (18% w/v in water) | as it suff. pH 5.6 |
| Dyestuff (blue CI 42080) | as it suffices |
| Water up to | 100 |

The obtained gel has a viscosity of 22,000 mPa.s and pH = 5.6.

EXAMPLE 4

The formulations prepared in the previous Examples are evaluated by the above test to determine the per cent increase of the peeling rate in vivo, calculated by the above formula. The results obtained at the fourth reading (12th day from the beginning of the experiment) and at the tenth reading (one month from the beginning of the experiment) have been reported in Table 1. The results are reported in Table 1.

The Table shows that the formulations for topical use containing the perfluoropolyether phosphate (Examples 1 and 2) both show a cutaneous peeling activity higher than that of the formulation with the same concentration of glycolic acid (Example 3 comparative). Furthermore the activity of the formulations of the present invention, differently from that with glycolic acid, is maintained in the time.

Table 1 shows also that, in the experimentation in vivo, the formulation containing PFPE phosphate pH 5.6 resulted more active, at both the observation times, in comparison with that containing PFPE phosphate pH 2.9.

EXAMPLE 5

Preparation of a composition for topical use in the gel form containing 5% of perfluoropolyether phosphate acid

Following the same procedure described in the Example 1, a gel having the following composition, in per cent by weight, is prepared:

| | |
|---|---|
| Perfluoropolyether phosphate acid (Fomblin® HC/P2-1000) | 5.0 |
| Ethanol | 15.0 |
| Xanthan rubber (Rodicare® T by Rhodia) | 0.8 |
| Water up to | 100 |

The obtained gel has pH = 2.5.

EXAMPLE 6 (comparative)

Preparation of a gel containing 2% of glycolic acid

By using the same procedure described in the Example 3 (comparative), a gel having the following composition, in per cent by weight, is prepared:

| | |
|---|---|
| Glycolic acid | 2.0 |
| Ethanol | 10.0 |
| Xanthan rubber (Rodicare® T by Rhodia) | 0.8 |
| Water up to | 100 |

The obtained gel has a pH = 2.6.

EXAMPLE 7

The cutaneous irritation test has been carried out by using the formulation of the Example 5 according to the present invention, containing 5% by weight of perfluoropolyether phosphate acid and having pH 2.5, and according to the Example 6 (comparative), containing 2% of glycolic acid pH 2.6.

The obtained results, expressed as mean irritation index, are reported in Table 2.

The results show that, with substantial equality of acid pH, the formulation of the present invention, having a concentration more than double (5% by weight) compared with the formulation of the comparative Example containing glycolic acid (2% by weight), does not show any irritating effect. On the contrary the latter shows cutaneous irritability.

EXAMPLE 8

Preparation of a batch of a composition for topical use in the gel form containing 5% of perfluoropolyether phosphate sodium salt

A determined amount (50 g) of bifunctional perfluoropolyether phosphate acid (PFPE phosphate) (Fomblin® HC/P2-1000), having number average molecular weight 2,500 in acid form, is dissolved at room temperature and under stirring in ethanol (100 g), then it is diluted with water previously heated at 85°C at about 800 g. A viscosifying agent (xanthan rubber, Rhodicare T, Rhodia SA, France) (10 g) is added by using a turbomixer. pH is corrected to 7.1 by slowing adding a sodium hydroxide aqueous solution. The composition is then rapidly cooled to room temperature by the aid of a water bath. Water at room temperature is added to yield 1 kg. The thus obtained gel has the following composition, as per cent by weight:

| | |
|---|---|
| Perfluoropolyether phosphate acid (Fomblin® HC/P2-1000) | 5.0 |
| Ethanol | 10.0 |
| Xanthan rubber (Rodicare® T by Rhodia) | 1.0 |
| Water up to | 100 |

The obtained gel has a viscosity of 2,600 mPa.s and pH = 7.1. The above prepared gel amount filled 32 70 ml tubes.

EXAMPLE 9

Preparation of a batch of a composition for topical use in the gel form containing 5% of perfluoropolyether phosphate acid

Example 8 is repeated but omitting the neutralization step with sodium hydroxide.
The thus obtained gel has the following composition, as per cent by weight:

| | |
|---|---|
| Perfluoropolyether phosphate acid (Fomblin® HC/P2-1000) | 5.0 |
| Ethanol | 10.0 |
| Xanthan rubber (Rodicare® T by Rhodia) | 1.0 |
| Water up to | 100 |

The obtained gel has a viscosity of 3,000 mPa.s and pH = 3.0. The above prepared gel amount filled 37 70 ml tubes.

EXAMPLE 10 (comparative)

Preparation of a batch of a composition for topical use in the gel form containing 5% of glycolic acid

Example 3 is repeated but using 71,5 g of a 70% by w aqueous solution of glycolic acid, instead of Fomblin®, 100 g of ethanol and diluting to 1 Kg with water. pH was corrected to 3.0. 1 Kg of gel was obtained, having the following composition, as per cent by weight:

| | |
|---|---|
| Glycolic acid | 5.0 |
| Ethanol | 10.0 |
| Xanthan rubber (Rodicare® T by Rhodia) | 1.0 |
| Water up to | 100 |

The obtained gel has a viscosity of 2,900 mPa.s and pH = 3.0. The above prepared gel amount filled 40 70 ml tubes.

EXAMPLE 11

The test of cutaneous irritation in vivo in occlusive conditions, described under "Methods", has been carried out on a group of 20 volunteers, applying the formulations of examples 8, 9 and 10 comparative as described in the test.

The results obtained are reported in Table 3. The difference of the results obtained with the gel of ex. 10 comparative are highly significant (p<0.01) v those of both gels of ex. 8 and ex. 9.

This experiment confirms that in the patch test the gel containin glycolic acid is more irritant than the gels of the present invention, the concentration of the active ingredient being the same.

Example 12

Erythema Value determination in vivo.

The test described under "Methods" has been carried out using the formulations prepared in examples 8, 9 and 10 comparative + a gel blank, not containing the active ingredient, but having the same compositions as for the other components.

The Erythema Value was determined on day 0, then on the 7th, 14th and 21th day (end of the experiment).

On day 0 it was found that the Erythema Values for the compositions of examples 8, 9 and 10 comparative of were not statistically different, as expected.

The results obtained on days 7, 14 and 21 are reported in Table 4. At any of said days the Erythema Value found in the sites of the volunteers' forearms treated with the formulation of example 10 comparative was higher than the sites treated with the compositions of examples 8 and 9.

The difference between the Erythema Value given by the sites treated with the formulation of example 10 comparative versus those treated with the formulations according to the present invention was statistically significant (p< 0.01).

Table 1

| Determination test of the per cent increase of the peeling rate in vivo: evaluations of the results at the fourth reading (twelfth day) and at the tenth and last reading (thirtieth day). The applied formula is that reported in the Test description (METHODS). The compositions contain the same amount by weight (5%) of active ingredient. | | |
|---|---|---|
| Compositions | readings | |
| | fourth % | tenth % |
| Ex. 1 Formulation PFPE phosphate pH 2.9 | 16.7 | 66.3* |
| Ex. 2 Formulation PFPE phosphate pH 5.6 | 33.2 | 83.5** |
| Ex. 3 (comparative) Glycolic acid pH 3.0 | 8.2 | 0 |
| Ex. 3A (comparative) Reference control | 0 | 0 |
| * one site out of three has given score = 0 (absence of fluorescence) ** two sites out of three have given score = 0 | | |

Table 2

| Determination of the mean cutaneous irritation index of the gel of the Example 5, having pH = 2.5 and containing 5% by weight of perfluoropolyether phosphate acid and of the gel of the Example 6 (comparative) having pH = 2.6 and containing 2% by weight of glycolic acid | | | |
|---|---|---|---|
| Example | Mean irritation index | | Classification |
| | after 15 minutes | after 24 hours | |
| Ex. 5 | 0.15 | 0.15 | non irritating |
| Ex. 6 Comp | 1.40 | 1.05 | slightly irritating |

EP 1 762 273 A2

Table 3

| Ex. 11: determination of the mean cutaneous irritation index of the gel of the Example 8, having pH = 3.0, of Example 9 having pH 7.1 and of Example 10 comparative, containing glycolic acid, having ph 3.0. The tested gels had the same concentration of the active ingredient (5 % by w.) | | |
|---|---|---|
| Example | Mean irritation index | |
| | after 15 minutes | after 24 hours |
| Ex. 8 | 0.25 | 0.25 |
| Ex. 9 | 0.20 | 0.20 |
| Ex. 10 Comp | 1.15 | 1.15 |

Table 4

| Ex. 12 : determination on days 7, 14 and 21 of the mean Erythema Value of the gels of Examples 8, 9 and 10 comparative applied on sites on the foreskin of volunteers as described under "Methods". | | | |
|---|---|---|---|
| Example | Mean Erythema Value | | |
| | after 7 days | after 14 days | after 21 days |
| Ex. 8 | 6.12 | 9.20 | 13.80 |
| Ex. 9 | 6.15 | 6.60 | 6.23 |
| Ex. 10 Comp | 10.86 | 16.23 | 19.78 |

**Claims**

1. Use for obtaining the cutaneous peeling of formulations comprising a component A):

(per)fluoropolyether phosphate of general formula:

$$R_f\text{-}[CF_2CH_2\text{-}O\text{-}L\text{-}P(O)(OZ_1)(OZ_2)]_l \qquad (I)$$

wherein $l = 1$ or 2;

- L is a bivalent linking group, preferably of the $(CHR_1CHR_2O)_n$ type wherein $R_1$, $R_2$ equal to or different from each other, are selected from H, $CH_3$; n is an integr between 1 and 50, preferably between 1 and 6;
- $Z_1$, equal to or different from $Z_2$, is selected from H, alkaline or ammonium cation; mono- di- or tri-alkanolammonium cation wherein the alkanol comprises from 1 to 20 C atoms, preferably 2-6 C atoms; di- or tri- or tetra-alkylammonium cation wherein the alkyl comprises from 1 to 20 C atoms, preferably 2-6 C atoms, or $R_f\text{-}(CF_2CH_2\text{-}O\text{-}L\text{-}$;
- $R_f$ represents a (per)fluoropolyether chain with a free valence when $l = 1$ and two free valences when $l = 2$, said (per)fluoropolyether chain having number average molecular weight in the range from about 400 to about 3,500, preferably from 800 to 2,000, said (per)fluoropolyether chain comprising repeating units selected from one or more of the following:

a) $-(C_3F_6O)\text{-}$;
b) $-(CF_2CF_2O)\text{-}$;
c) $-(CFL_0O)\text{-}$, wherein $L_0 = \text{-F, -CF}_3$;
d) $-CF_2(CF_2)_{z'}CF_2O\text{-}$, wherein z' is an integer 1 or 2;
e) $-CH_2CF_2CF_2O\text{-}$.

2. Use according to claim 1, wherein $R_f$ is monofunctional ($l = 1$), and the end group is of the perfluoroalkyl type, preferably $CF_3O\text{-}$, $C_2F_5O\text{-}$, $C_3F_7O\text{-}$, wherein optionally one fluorine atom can be substituted by one chlorine or hydrogen atom.

13

3. Use according to claims 1-2, wherein in formula (I) $Z_1$ or $Z_2$ are different from $R_f$-[-$CF_2CH_2$-O-L-; preferably $Z_1 = Z_2$ = H and l = 2.

4. Use according to claims 1-3, wherein $R_f$ is a bifunctional (per)fluoropolyether substituent and preferably has one of the following structures:

1) -$(CF_2O)_a$-$(CF_2CF_2O)_b$-
a and b being integers such to give the above number average molecular weight, b/a being between 0.3 and 10, extremes included, a being different from 0;
2) -$(CF_2$-$(CF_2)_{z'}$-$CF_2O)_{b'}$-
b' being an integer and such to give the above number average molecular weight, z' being an integer and equal to 1 or 2;
3) -$(C_3F_6O)_r$-$(C_2F_4O)_b$-$(CFL_0O)_t$-,
r, b and t being integers such to give the above number average molecular weight, r/b = 0.5-2.0 when b is different from 0, (r+b)/t = 10-30, t being different from 0;
4) -$(OC_3F_6)_r$-$(CFL_0O)_t$-$OCF_2$-$R'_f$-$CF_2O$-$(C_3F_6O)_r$-$(CFL_0O)_t$-
r and t being integers as above such to give the above number average molecular weight;
5) -$(CF_2CF_2CH_2O)_{q'}$-$R'_f$-O-$(CH_2CF_2CF_2O)_{q'}$-
wherein:

q' is an integer such to give the above number average molecular weight;
$R'_f$ is a fluoroalkylene group from 1 to 4 carbon atoms;
$L_0$ is selected from F, $CF_3$;

6) -$(C_3F_6O)_r$-$OCF_2$-$R'_f$-$CF_2O$-$(C_3F_6O)_r$-
wherein r is an integer such to give the above number average molecular weight and $R'_f$ is as above.

5. Use according to claim 4, wherein the unit -$(C_3F_6O)$- represents units of formula: -$(CF(CF_3)CF_2O)$- and/or -$(CF_2CF(CF_3)O)$-.

6. Use according to claims 1-5, wherein in formula (I) the (per)fluoropolyether chain $R_f$:
when l = 2 is selected from the following structures:

-$(CF_2O)_a$-$(CF_2CF_2O)_b$-;
-$(C_3F_6O)_r$-$(C_2F_4O)_b$-$(CFL_0O)_t$-;

preferably $R_f$ = -$(CF_2O)_a$-$(CF_2CF_2O)_b$- (l = 2);
when l = 1, $R_f$ = -$(C_3F_6O)_r$-$(CFL_0O)_t$-;
wherein $L_0$ and the a, b, r, t indexes have the above value.

7. Use according to claims 1-6, wherein in formula (I) L = $(CH_2$-$CH_2O)_n$ n being an integer from 1 to 4; $Z_1$, equal to or different from $Z_2$, is selected from H, $NH_4$, or an alkaline metal cation; l = 2.

8. Use according to claim 7, wherein the compounds of formula (I) are selected from the following:

$$CF_3\text{-}O(CF_2CF(CF_3)O)_r(CF_2O)_a\text{-}CF_2\text{-}CH_2(OCH_2CH_2)_nO\text{-}PO(OH)_2 \quad\quad (II)$$

wherein r/a = 0.5-2.0 (a being different from 0) and n = 1-2;

$$[\text{-}O(CF_2CF_2O)_b(CF_2O)_a\text{-}]\,[\text{-}CF_2\text{-}CH_2\text{-}\,(OCH_2CH_2)_nO\text{-}PO(OH)_2]_2 \quad\quad (III)$$

wherein b/a = 0.5-3.0 (a being different from 0) and n = 1-2;
a, b and r have the above meaning.

9. Use according to claims 1-8, wherein the concentration of component A) in the formulations is between 0.1% and 30% by weight, preferably between 1% and 10% by weight.

10. Use according to claims 1-9, wherein the compositions comprise component A) solubilized in water (component C)) or in a polar organic solvent (component B)), or their mixtures.

**11.** Use according to claim 10, wherein the polar organic solvent is selected from one or more of the following:

  - linear or branched when possible alcohols, from 2 to 3 carbon atoms and their ethers, preferably methylic alcohols;
  - linear or branched glycols from 2 to 6 carbon atoms; optionally mono alkyletherified wherein the alkyl group, linear or banched, is $C_1$-$C_4$;
  - ethers as, for example, dimethoxymethane;
  - esters of acids and alcohols having, respectively from 1 to 4 carbon atoms and being liquid at room temperature; or related mixtures.

**12.** Use according to claims 10-11, wherin the water (component C)), can be used alone or in admixture with a solvent B).

**13.** Use according to claims 10-12, wherein, when water (component C)) is used for preparing the solution of A) in C), a basic component is added to salify at least 60% of the acid groups present and obtain a final pH not lower than 4.

**14.** Use according to claims 10-13, wherein mixtures of water with at least a solvent B) are used for preparing the solution of A) in the mixture of C) and B), preferably component A) is dissolved in solvent B) before adding water (component C)).

**15.** Use according to claims 1-14, wherein the formulations are used in the form of gels and emulsions.

**16.** Use according to claims 1-15, whereien the formulations optionally contain additives selected from emollient, emulsifying, viscosifying, hydrating, preserving agents, anti-ageing agents as, for example, sun filters, antioxidant agents; peeling agents.

**17.** Use according to claims 1-16, wherein the compositions are prepared by dissolving component A) in a solvent or mixture of solvents comprising B) or C) or their mixtures, by optionally adding further components and/or excipients, obtaining the percentage by weight of component A) comprised in the above limits.

**18.** Use according to claims 10-17, wherein component B) is selected from the following: ethanol, ehtylene glycol, isopropanol, propanol, acetone, methoxyethanol, propylen glycol, propan-1,2-diol, dimethoxymethane, methoxy-isopropanol, diethylen glycol, butan-1,4-diol, diethylenglycolmonoethylenether, pentan-1,2-diol, diethylenglycol monoethylether, dipropylenglycol, dipropylenglycol monomethylether and dipropylenglycol monoethylether; more preferably ethanol or pentan-1,2-diol are used.

**19.** Use according to claims 1-18, wherein the compositions do not contain surfactants/emulsifiers and are in the form of gel.

**20.** Use according to claims 1-18, wherein the compositions are in the form of emulsions and the amount of surfactants/emulsifiers ranges from 0% to 5% by weight on the whole composition.

**21.** Use according to claims 1-20, wherein component A) is in admixture with one or more compounds having a peeling activity.

**22.** Use according to claim 21, wherein the compounds having a peeling activity are selected from alpha hydroxyacids, beta hydroxyacids, ketoacids, poly-alpha-hydroxyacids, non hydroxylated carboxylic organic acids having a short chain $C_2$-$C_4$, retinol.

**23.** Use according to claim 22, wherein the compounds having peeling activity are selected from the following: lactic acid, glycolic acid, salicylic acid, trichloroacetic acid, acetic acid, hydroxyacetic acid, piruvic acid, citric acid, maleic acid, malic acid, tartaric acid, ascorbic acid, polylactic acid, hydroxyoctanoic acid, tartronic acid, glyceric acid, gluconic acid, glucuronic acid, mandelic acid, benzylic acid, 2-hydroxybutyric acid.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3665041 A
- US 2242218 A
- US 3715378 A
- EP 239123 A
- EP 148482 A
- US 3810874 A

### Non-patent literature cited in the description

- International Cosmetic Ingredient Dictionary and Handbook. The Cosmetic, Toiletry, and Fragrance Association publisher, 1997
- **E.J. VAN SCOTT et al.** Hyperkeratinization, Corneocyte Cohesion, and Alpha Hydroxy Acids. *J. Am. Acad. Dermatol.,* 1984, vol. 11, 867-879
- **E.J. VAN SCOTT.** Control of Keratinization with $\alpha$ Hydroxy Acids and related Compounds. *Acta Dermatol.,* October 1994, vol. 110, 586-590
- **MARKS R. et al.** *J. Dermatol.,* 1984, vol. 111, 265-270
- **B.D. RIDGE et al.** *J. Dermatol.,* 1988, vol. 118, 164-174